# EUROPEAN PATENT APPLICATION

(11) **EP 4 464 450 A1**
(43) Date of publication of application: **20.11.2024**
(21) Application number: 24174202.2
(22) Date of filing: 03.05.2024
(51) Int. Cl.: B23K 20/02, G01N 29/30

(54) **REFERENCE STANDARDS COMPRISING KNOWN DEFECTS AND METHODS FOR MANUFACTURING SUCH REFERENCE STANDARDS**

(30) Priority: 03.05.2023 US 202363499781 P; 20.09.2023 US 202318470575
(71) Applicant: The Boeing Company, Arlington, VA 22202 (US)
(72) Inventor: WILSON, Paul N., Arlington, 22202 (US); PECINA, Joseph A., Arlington, 22202 (US); NWOKE, Dominic, Arlington, 22202 (US); HAWORTH, Troy A., Arlington, 22202 (US); NEMATOLLAHI, Mohammedreza, Arlington, 22202 (US)
(74) Representative: Bryn-Jacobsen, Caelia

(57) **Abstract**

Reference standards that include known defects for use in nondestructive inspection are disclosed. In one example, the reference standard includes a metallic body, a another metallic body and a predetermined quantity of a contaminant material. The metallic bodies are diffusion bonded along a seamless interface. The predetermined quantity of a contaminant material has a predetermined composition positioned at a predetermined location proximate the seamless interface. Methods for manufacturing the reference standards are also disclosed. In one example, a method for manufacturing the reference standard includes positioning a predetermined quantity of a contaminant material having a predetermined composition between a bonding surface of a metallic body and another bonding surface of another metallic body. The two bonding surface are diffusion bonded to yield a bonded article (e.g., a reference standard).

## Description

### PRIORITY

This application claims priority from U.S. Ser. No. 63/499,781 filed on May 3, 2023.

### FIELD

The present disclosure relates to nondestructive inspection of metallic articles and, more particularly, to reference standards that include known defects that may be used in connection with nondestructive inspection of metallic articles.

### BACKGROUND

Titanium metal injection molding (MIM) is a replacement fabrication method for machining parts from titanium plate to reduce costs and material waste. To use MIM for titanium parts, the parts must be inspected differently than current techniques. New nondestructive inspection (NDI) standards are needed. There are currently no NDI standards for internal void and porosity defects on consolidated powder metallurgy parts. Moreover, current methods for manufacturing reference standards for NDI generally utilize drilling material out of a section, then refilling with a plug containing the defect. These techniques are fraught with alignment, tolerance and gapping issues that can interfere with a clean radiographic signal. Other existing methods use defects open to the surface. These methods are not properly representative of the behavior of defects internal to the material.

CT scanning is also used to detect and locate defects within consolidated titanium powder metallurgy parts. However, use of CT technology is not conducive for production inspection due to the high system and labor cost, operator involvement in the analysis and data storage requirements. For production inspection a go/no-go disposition is needed. CT scanning is useful in the lab but requires a long acquisition time to attain the needed resolution to resolve small defects of interest at the limit of acceptance and is not practical for production inspection at a high rate. Currently there are no generic CT established standards with void defects at, below and/or above the specified limit of acceptance to validate positive and/or negative indications.

Accordingly, those skilled in the art continue with research and development efforts to improve the design of reference standards for NDI of metallic articles.

### SUMMARY

Disclosed are examples of reference standards with known defects for use in nondestructive testing and methods for manufacturing such reference standards. The following is a non-exhaustive list of examples, which may or may not be claimed, of the subject matter according to the present disclosure.

In an example, the disclosed method for manufacturing a reference standard that includes a known defect includes: (1) positioning a predetermined quantity of a contaminant material having a predetermined composition between a first bonding surface of a first metallic body and a second bonding surface of a second metallic body; and (2) diffusion bonding the first bonding surface of the first metallic body to the second bonding surface of the second metallic body to yield a bonded article.

In an example, the disclosed reference standard for use in nondestructive inspection includes a first metallic body, a second metallic body and a predetermined quantity of a contaminant material. The second metallic body diffusion bonded to the first metallic body along a seamless interface. The predetermined quantity of a contaminant material having a predetermined composition positioned at a predetermined location proximate the seamless interface.

In another example, the disclosed method for manufacturing a reference standard that includes a known defect utilizes a first metallic body with a first bonding surface and a second metallic body with a second bonding surface. In this example, the method includes: (1) forming a recess in the first bonding surface of the first metallic body; (2) positioning the second bonding surface of the second metallic body in intimate contact with the first bonding surface; and (3) diffusion bonding the first bonding surface of the first metallic body to the second bonding surface of the second metallic body to yield a bonded article.

In another example, the disclosed reference standard for use in nondestructive inspection includes a first metallic body, a second metallic body and a recess. The second metallic body diffusion bonded to the first metallic body along a seamless interface. The recess positioned at a predetermined location proximate the seamless interface.

In yet another example, the disclosed method for manufacturing a reference standard that includes a known defect includes: (1) incorporating a predetermined quantity of a contaminant material having a predetermined composition into a metallic powder composition to yield a contaminated metallic powder composition; (2) shaping the contaminated metallic powder composition to yield a shaped metallic powder composition; and (3) sintering the shaped metallic powder composition to yield a consolidated article.

Other examples of the disclosed reference standards and methods for manufacturing such reference standards will become apparent from the following detailed description, the accompanying drawings and the appended claims.

### BRIEF DESCRIPTION OF THE DRAWINGS

Fig. 1 is a flow diagram of an example of a method for manufacturing a reference standard that includes a known defect;
Figs. 2A-C show three stages of a reference standard for use in nondestructive inspection during its manufacture;
Fig. 3 is a flow diagram of another example of a method for manufacturing a reference standard that includes a known defect;
Figs. 4A-D show four stages of another reference standard for use in nondestructive inspection during its manufacture;
Fig. 5 is a flow diagram of yet another example of a method for manufacturing a reference standard that includes a known defect;
Fig. 6 is a functional flow diagram of an example of a metal injection molding process for manufacturing a reference standard that includes a known defect;
Fig. 7 is a block diagram of aircraft production and service methodology; and
Fig. 8 is a schematic illustration of an aircraft.

### DETAILED DESCRIPTION

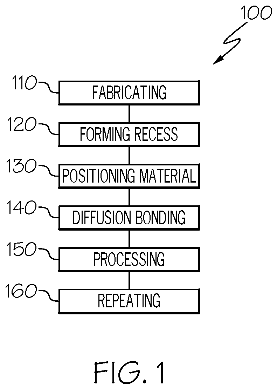

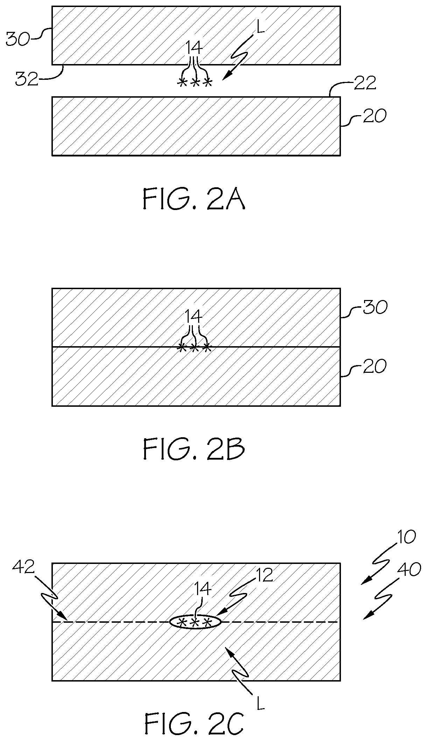

Referring generally to Figs. 1 and 2A-C, by way of examples, the present disclosure is directed to a method 100 for manufacturing a reference standard 10 that includes a known defect 12. Fig. 1 discloses an example of the method 100 that includes positioning 130 a contaminant material 14 between first and second bonding surfaces 22, 32 and diffusion bonding 140 the first and second bonding surfaces 22, 32. Fig. 2A discloses a first stage of the reference standard 10 in relation to the positioning 130. Fig. 2B discloses a second stage of the reference standard 10 in relation to the diffusion bonding 140. Fig. 2C discloses a third stage of the reference standard 10 as a bonded article 40 resulting from the method 100.

With continued reference to Figs. 1 and 2A-C, in one or more examples, a method 100 for manufacturing a reference standard 10 that includes a known defect 12 is disclosed. The method 100 includes positioning 130 a predetermined quantity of a contaminant material 14 having a predetermined composition between a first bonding surface 22 of a first metallic body 20 and a second bonding surface 32 of a second metallic body 30. At 140, the first bonding surface 22 of the first metallic body 20 is diffusion bonded to the second bonding surface 32 of the second metallic body 30 to yield a bonded article 40.

In another example of the method 100, the contaminant material 14 has a contaminant material density and the first metallic body 20 has a bulk density. In this example, the contaminant material density is substantially less than the bulk density.

In yet another example of the method 100, the contaminant material 14 includes a carbonaceous material.

In still another example of the method 100, the contaminant material 14 includes graphite.

In still yet another example of the method 100, the first metallic body 20 includes a titanium alloy. In this example, the second metallic body 30 includes the titanium alloy and the contaminant material 14 includes graphite.

In another example of the method 100, the contaminant material 14 has a contaminant material density and the first metallic body 20 has a bulk density. In this example, the contaminant material density is substantially greater than the bulk density.

In yet another example of the method 100, the contaminant material 14 includes niobium, molybdenum, tantalum, tungsten, rhenium or any other suitable contaminant material in any suitable combination.

In still another example of the method 100, the contaminant material 14 includes tungsten.

In still yet another example of the method 100, the first metallic body 20 includes a titanium alloy. In this example, the second metallic body 30 includes the titanium alloy and the contaminant material 14 includes tungsten.

In another example of the method 100, the positioning 130 the predetermined quantity of the contaminant material 14 includes positioning the predetermined quantity of the contaminant material 14 at a predetermined location (L) between the first bonding surface 22 of the first metallic body 20 and the second bonding surface 32 of the second metallic body 30.

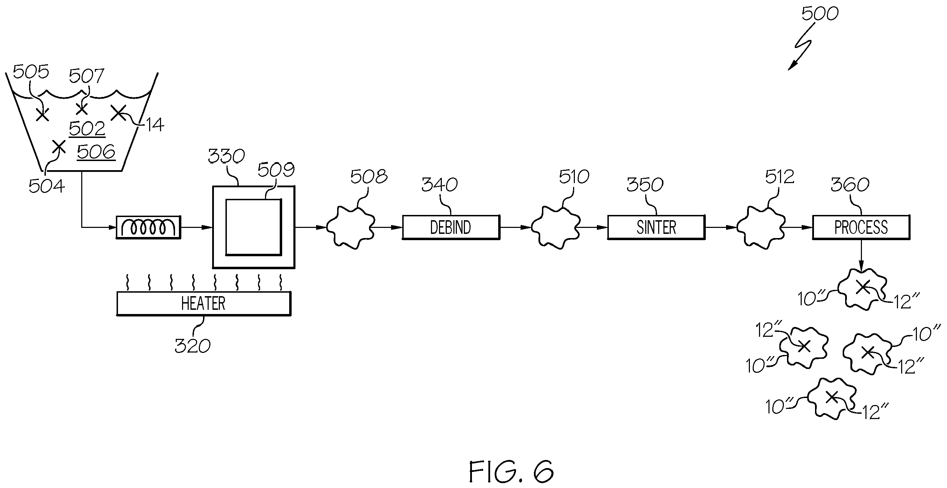

In yet another example, the method 100 also includes fabricating 110 the first metallic body 20, the second metallic body 30 or both the first and second metallic body 20, 30 from a metallic powder feedstock 502 (see Fig. 6) by metal injection molding 500 (see Fig. 6).

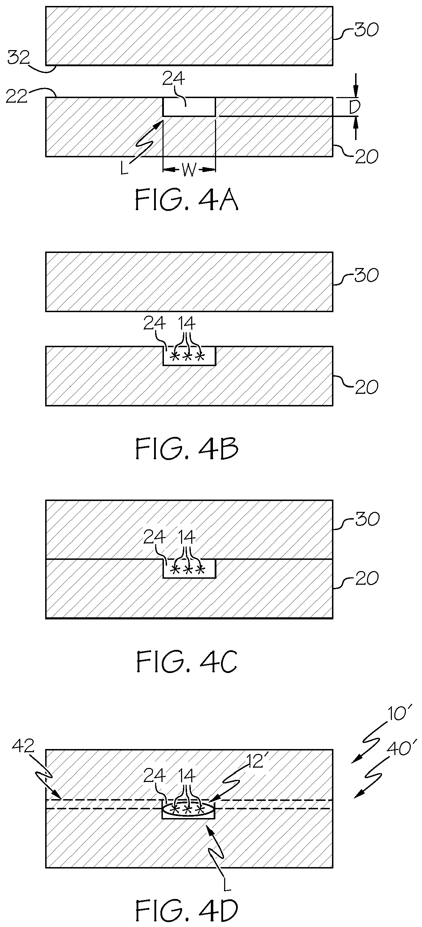

In still another example, the method 100 also includes forming 120 a recess 24 (see Fig. 4A) in the first bonding surface 22 of the first metallic body 20. In this example, the positioning 130 the predetermined quantity of the contaminant material 14 includes positioning the predetermined quantity of the contaminant material 14 in the recess 24.

In still yet another example of the method 100, the first bonding surface 22 and the second bonding surface 32 are configured to meet in intimate contact.

In another example, after the diffusion bonding 140, the method 100 also includes processing 150 the bonded article 40. In a further example, the processing 150 includes hot isostatic pressing, cold isostatic pressing, machining, heat-treating, plating and/or any other suitable type of processing in any suitable combination.

In another example, the method 100 also includes repeating 160 the positioning 130 and the diffusion bonding 140 to manufacture a plurality of reference standards 10. In this example, each reference standard 10 of the plurality of reference standards 10 includes a unique known defect 12.

With reference again to Figs. 2A-C, in one or more examples, a reference standard 10 for use in nondestructive inspection includes a first metallic body 20, a second metallic body 30 and a predetermined quantity of a contaminant material 14. The second metallic body 30 diffusion bonded to the first metallic body 20 along a seamless interface 42. The predetermined quantity of a contaminant material 14 having a predetermined composition positioned at a predetermined location (L) proximate the seamless interface 42.

In another example of the reference standard 10, the contaminant material 14 is completely surrounded by the first metallic body 20 and the second metallic body 30.

In yet another example of the reference standard 10, the first metallic body 20 includes a titanium alloy. In this example, the second metallic body 30 includes the titanium alloy and the contaminant material 14 includes graphite.

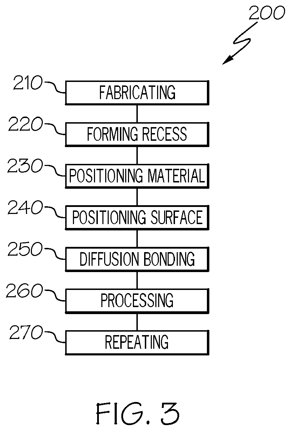

Referring generally to Figs. 3 and 4A-D, by way of examples, the present disclosure is directed to a method 200 for manufacturing a reference standard 10' that includes a known defect 12'. Fig. 3 discloses an example of the method 200 that includes forming 220 a recess 24 in a first bonding surface 22, positioning 240 a second bonding surface 32 in intimate contact with the first bonding surface 22, and diffusion bonding 250 the first and second bonding surfaces 22, 32. Fig. 4A discloses a first stage of the reference standard 10' in relation to the forming 220. Fig. 4B discloses a second stage of the reference standard 10 in relation to the positioning 230 a contaminant material 14 within the recess 24. Fig. 4C discloses a third stage of the reference standard 10' in relation to the diffusion bonding 250. Fig. 4D discloses a third stage of the reference standard 10' as a bonded article 40' resulting from the method 200.

With continued reference to Figs. 3 and 4A-D, in one or more examples, a method 200 for manufacturing a reference standard 10' that includes a known defect 12' is disclosed. The method 200 utilizes a first metallic body 20 with a first bonding surface 22 and a second metallic body 30 with a second bonding surface 32. The method 200 includes forming 220 a recess 24 in the first bonding surface 22 of the first metallic body 20. At 240, the second bonding surface 32 of the second metallic body 30 is positioned in intimate contact with the first bonding surface 22. At 250, the first bonding surface 22 of the first metallic body 20 is diffusion bonded to the second bonding surface 32 of the second metallic body 30 to yield a bonded article 40'.

In another example of the method 200, the forming 220 the recess 24 includes forming a recess 24 having a predefined size.

In yet another example of the method 200, the forming 220 the recess 24 includes forming a recess 24 having a predefined shape.

In still another example of the method 200, the forming 220 the recess 24 includes forming the recess 24 at a predetermined location (L) on the first bonding surface 22 of the first metallic body 20.

In still yet another example of the method 200, the forming 220 the recess 24 includes forming a recess 24 having a maximum width (W) and a maximum depth (D). In this example, the maximum width (W), the maximum depth (D) or both the maximum width (W) and depth (D) fall within a range spanning from 0.001 inches to 0.060 inches or any other suitable distance range.

In another example of the method 200, the forming 220 the recess 24 includes drilling the first metallic body 20, punching the first metallic body 20, scratching the first metallic body 20, or any other suitable forming technique in any suitable combination.

In yet another example, the method 200 also includes fabricating 210 the first metallic body 20, the second metallic body 30 or both the first and second metallic body 20, 30 from a metallic powder feedstock 502 (see Fig. 6) by metal injection molding 500 (see Fig. 6).

In still another example, the method 200 also includes positioning 230 a predetermined quantity of a contaminant material 14 having a predetermined composition within said recess 24.

In a further example, the contaminant material 14 has a contaminant material density and the first metallic body 20 has a bulk density. In this example, the contaminant material density is substantially less than the bulk density.

In another further example, the contaminant material 14 includes a carbonaceous material.

In yet another further example, the contaminant material 14 includes graphite.

In still another further example, the first metallic body 20 includes a titanium alloy. In this example, the second metallic body 30 includes the titanium alloy and the contaminant material 14 includes graphite.

In still yet another further example, the contaminant material 14 has a contaminant material density and the first metallic body 20 has a bulk density. In this example, the contaminant material density is substantially greater than the bulk density.

In another further example, the contaminant material 14 includes niobium, molybdenum, tantalum, tungsten, rhenium, or any other suitable contaminant material in any suitable combination.

In yet another further example, the contaminant material 14 includes tungsten.

In still another further example, the first metallic body 20 includes a titanium alloy. In this example, the second metallic body 30 includes the titanium alloy and the contaminant material 14 includes tungsten.

In still yet another further example, the contaminant material 14 substantially fills the recess 24.

In another example, after the diffusion bonding 250, the method 200 also includes processing 260 the bonded article 40'. In a further example, the processing 260 includes hot isostatic pressing, cold isostatic pressing, machining, heat-treating, plating or any other suitable processing technique in any suitable combination.

In another example, the method 200 also includes repeating 270 the forming 220, the positioning 240, and the diffusion bonding 250 to manufacture a plurality of reference standards 10'. In this example, each reference standard 10'of the plurality of reference standards 10' includes a unique known defect 12'.

With continued reference to Figs. 4A-D, in one or more example, a reference standard 10' for use in nondestructive inspection includes a first metallic body 20, a second metallic body 30, and a recess 24. The second metallic body 30 diffusion bonded to the first metallic body 20 along a seamless interface 42. The recess 24 positioned at a predetermined location (L) proximate the seamless interface 42.

In another example of the reference standard 10', the recess 24 is completely surrounded by the first metallic body 20 and the second metallic body 30.

In yet another example, the reference standard 10' also includes a contaminant material 14 having a predetermined composition received in the recess 24.

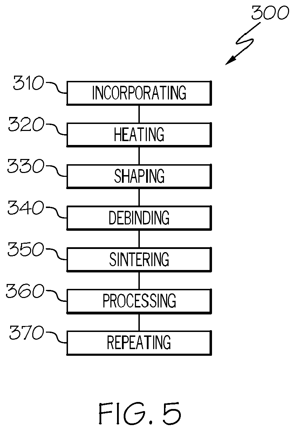

Referring generally to Figs. 5 and 6, by way of examples, the present disclosure is directed to a method 300 for manufacturing a reference standard 10" that includes a known defect 12". Fig. 5 discloses an example of the method 300 that includes incorporating 310 a contaminant material 14 into a metallic powder composition 504, shaping 330 the contaminated metallic powder composition 506, and sintering the shaped metallic powder composition 508. Fig. 6 discloses an example of metal injection molding 500.

With continued reference to Figs. 5 and 6, in one or more examples, a method 300 for manufacturing a reference standard 10" that includes a known defect 12" is disclosed. The method 300 includes incorporating 310 a predetermined quantity of a contaminant material 14 having a predetermined composition into a metallic powder composition 504 to yield a contaminated metallic powder composition 506. At 330, the contaminated metallic powder composition 506 is shaped to yield a shaped metallic powder composition 508. At 350, the shaped metallic powder composition 508 is sintered to yield a consolidated article 512.

In another example of the method 300, the incorporating 310 the predetermined quantity of the contaminant material 14 into the metallic powder composition 504 includes incorporating the predetermined quantity of the contaminant material 14 into a predetermined quantity of the metallic powder composition 504 to yield the contaminated metallic powder composition 506 having a known contaminant material concentration.

In yet another example of the method 300, the incorporating 310 the predetermined quantity of the contaminant material 14 into the metallic powder composition 504 includes dispersing the predetermined quantity of the contaminant material 14 throughout the metallic powder composition 504.

In still another example of the method 300, the contaminant material 14 has a contaminant material density and the metallic powder composition 504 has a bulk density. In this example, the contaminant material density is substantially less than the bulk density.

In still yet another example of the method 300, the contaminant material 14 includes a carbonaceous material.

In another example of the method 300, the contaminant material 14 includes graphite.

In yet another example of the method 300, the metallic powder composition 504 includes a titanium alloy. In this example, the contaminant material 14 includes graphite.

In still another example of the method 300, the contaminant material 14 has a contaminant material density and the metallic powder composition 504 has a bulk density. In this example, the contaminant material density is substantially greater than the bulk density.

In still yet another example of the method 300, the contaminant material 14 includes niobium, molybdenum, tantalum, tungsten, rhenium or any other suitable contaminant material in any suitable combination.

In another example of the method 300, the contaminant material 14 includes tungsten.

In yet another example of the method 300, the metallic powder composition 504 includes a titanium alloy. In this example, the contaminant material 14 includes tungsten.

In still another example of the method 300, the shaping 330 the contaminated metallic powder composition 506 includes injecting the contaminated metallic powder composition 506 into a mold cavity 509 to yield the shaped metallic powder composition 508. In a further example, the contaminated metallic powder composition 506 includes a binder 505 in admixture with the contaminant material 14 and a metallic powder 507. In another further example, the method 300 also includes heating 320 the contaminated metallic powder composition 506 during the shaping 330. In yet another further example, prior to the sintering 350, the method 300 also includes debinding 340 the shaped metallic powder composition 508.

In still yet another example, after the sintering 350, the method 300 also includes processing 360 the consolidated article 512. In a further example, the processing 360 includes hot isostatic pressing, cold isostatic pressing, machining, heat-treating, plating or any other suitable processing technique in any suitable combination.

In another example, the method 300 also includes repeating 370 the incorporating 310, the shaping 330, and the sintering 350 to manufacture a plurality of reference standards 10". Each reference standard 10" of the plurality of reference standards 10" including a unique known defect 12".

With continued reference to Figs. 5 and 6, in one or more examples, a reference standard 10" for use in nondestructive inspection is manufactured according to the method 300 for manufacturing the reference standard 10".

Examples of reference standards 10, 10' and 10" with known defects 12, 12' and 12" and methods 100, 200, 300 for manufacturing such reference standard 10, 10' and 10" may be related to or used in the context of aircraft manufacturing. Although an aircraft example is described, the examples and principles disclosed herein may be applied to other products in the aerospace industry and other industries, such as the automotive industry, the space industry, the construction industry and other design and manufacturing industries. Accordingly, in addition to aircraft, the examples and principles disclosed herein may apply to the use of reference standards with known defects for nondestructive inspection in the manufacture of various types of vehicles and in the construction of various types of buildings.

The preceding detailed description refers to the accompanying drawings, which illustrate specific examples described by the present disclosure. Other examples having different structures and operations do not depart from the scope of the present disclosure. Like reference numerals may refer to the same feature, element, or component in the different drawings. Throughout the present disclosure, any one of a plurality of items may be referred to individually as the item and a plurality of items may be referred to collectively as the items and may be referred to with like reference numerals. Moreover, as used herein, a feature, element, component, or step preceded with the word "a" or "an" should be understood as not excluding a plurality of features, elements, components, or steps, unless such exclusion is explicitly recited.

Illustrative, non-exhaustive examples, which may be, but are not necessarily, claimed, of the subject matter according to the present disclosure are provided above. Reference herein to "example" means that one or more feature, structure, element, component, characteristic, and/or operational step described in connection with the example is included in at least one aspect, embodiment, and/or implementation of the subject matter according to the present disclosure. Thus, the phrases "an example," "another example," "one or more examples," and similar language throughout the present disclosure may, but do not necessarily, refer to the same example. Further, the subject matter characterizing any one example may, but does not necessarily, include the subject matter characterizing any other example. Moreover, the subject matter characterizing any one example may be, but is not necessarily, combined with the subject matter characterizing any other example.

As used herein, a system, apparatus, device, structure, article, element, component, or hardware "configured to" perform a specified function is indeed capable of performing the specified function without any alteration, rather than merely having potential to perform the specified function after further modification. In other words, the system, apparatus, device, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware that enable the system, apparatus, structure, article, element, component, or hardware to perform the specified function without further modification. For purposes of this disclosure, a system, apparatus, device, structure, article, element, component, or hardware described as being "configured to" perform a particular function may additionally or alternatively be described as being "adapted to" and/or as being "operative to" perform that function.

Unless otherwise indicated, the terms "first," "second," "third," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

As used herein, the phrase "at least one of," when used with a list of items, means different combinations of one or more of the listed items may be used and only one of each item in the list may be needed. For example, "at least one of item A, item B, and item C" may include, without limitation, item A or item A and item B. This example also may include item A, item B, and item C, or item B and item C. In other examples, "at least one of' may be, for example, without limitation, two of item A, one of item B, and ten of item C; four of item B and seven of item C; and other suitable combinations. As used herein, the term "and/or" and the "j" symbol includes any and all combinations of one or more of the associated listed items.

As used herein, the terms "coupled," "coupling," and similar terms refer to two or more elements that are joined, linked, fastened, attached, connected, put in communication, or otherwise associated (e.g., mechanically, electrically, fluidly, optically, electromagnetically) with one another. In various examples, the elements may be associated directly or indirectly. As an example, element A may be directly associated with element B. As another example, element A may be indirectly associated with element B, for example, via another element C. It will be understood that not all associations among the various disclosed elements are necessarily represented. Accordingly, couplings other than those depicted in the figures may also exist.

As used herein, the term "approximately" refers to or represents a condition that is close to, but not exactly, the stated condition that still performs the desired function or achieves the desired result. As an example, the term "approximately" refers to a condition that is within an acceptable predetermined tolerance or accuracy, such as to a condition that is within 10% of the stated condition. However, the term "approximately" does not exclude a condition that is exactly the stated condition. As used herein, the term "substantially" refers to a condition that is essentially the stated condition that performs the desired function or achieves the desired result.

In Figs. 1, 3, 5 and 6, referred to above, the blocks may represent operations, steps, and/or portions thereof, and lines connecting the various blocks do not imply any particular order or dependency of the operations or portions thereof. It will be understood that not all dependencies among the various disclosed operations are necessarily represented. Figs. 1, 3, 5 and 6 and the accompanying disclosure describing the operations of the disclosed methods set forth herein should not be interpreted as necessarily determining a sequence in which the operations are to be performed. Rather, although one illustrative order is indicated, it is to be understood that the sequence of the operations may be modified when appropriate. Accordingly, modifications, additions and/or omissions may be made to the operations illustrated and certain operations may be performed in a different order or simultaneously. Additionally, those skilled in the art will appreciate that not all operations described need be performed.

Figs. 2A-C, 4A-D and 6, referred to above, may represent functional elements, features, or components thereof and do not necessarily imply any particular structure. Accordingly, modifications, additions and/or omissions may be made to the illustrated structure. Additionally, those skilled in the art will appreciate that not all elements, features, and/or components described and illustrated in Figs. 2A-C, 4A-D and 6, referred to above, need be included in every example and not all elements, features, and/or components described herein are necessarily depicted in each illustrative example. Accordingly, some of the elements, features, and/or components described and illustrated in Figs. 2A-C, 4A-D and 6 may be combined in various ways without the need to include other features described and illustrated in Figs. 2A-C, 4A-D and 6, other drawing figures, and/or the accompanying disclosure, even though such combination or combinations are not explicitly illustrated herein. Similarly, additional features not limited to the examples presented, may be combined with some or all the features shown and described herein. Unless otherwise explicitly stated, the schematic illustrations of the examples depicted in Figs. 2A-C, 4A-D and 6, referred to above, are not meant to imply structural limitations with respect to the illustrative example. Rather, although one illustrative structure is indicated, it is to be understood that the structure may be modified when appropriate. Accordingly, modifications, additions and/or omissions may be made to the illustrated structure. Furthermore, elements, features, and/or components that serve a similar, or at least substantially similar, purpose are labeled with like numbers in each of Figs. 2A-C, 4A-D and 6, and such elements, features, and/or components may not be discussed in detail herein with reference to each of Figs. 2A-C, 4A-D and 6. Similarly, all elements, features, and/or components may not be labeled in each of Figs. 2A-C, 4A-D and 6, but reference numerals associated therewith may be utilized herein for consistency.

Further, references throughout the present specification to features, advantages, or similar language used herein do not imply that all the features and advantages that may be realized with the examples disclosed herein should be, or are in, any single example. Rather, language referring to the features and advantages is understood to mean that a specific feature, advantage, or characteristic described in connection with an example is included in at least one example. Thus, discussion of features, advantages, and similar language used throughout the present disclosure may, but does not necessarily, refer to the same example.

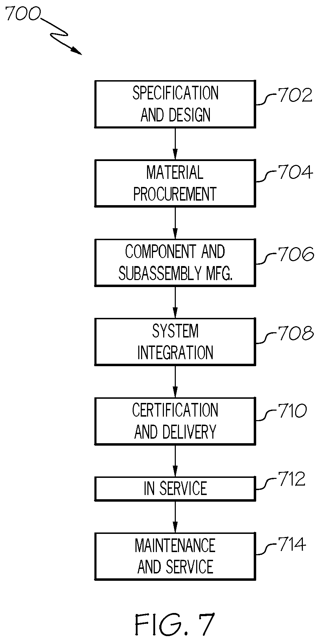

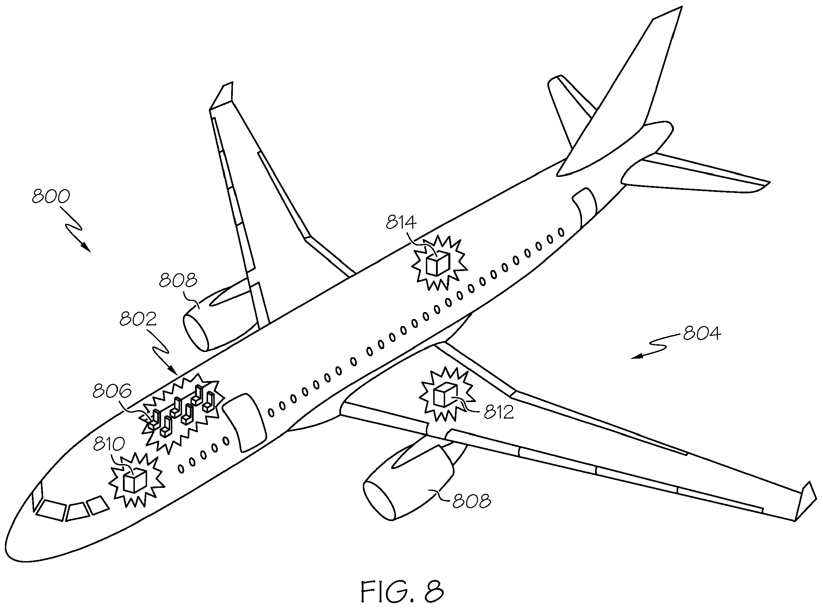

Examples of the subject matter disclosed herein may be described in the context of aircraft manufacturing and service method 700 as shown in Fig. 7 and aircraft 800 as shown in Fig. 8. In one or more examples, the disclosed methods and systems for associating test data for a part under test with an end item coordinate system may be used in aircraft manufacturing. During pre-production, the service method 700 may include specification and design (block 702) of aircraft 800 and material procurement (block 704). During production, component and subassembly manufacturing (block 706) and system integration (block 708) of aircraft 800 may take place. Thereafter, aircraft 800 may go through certification and delivery (block 710) to be placed in service (block 712). While in service, aircraft 800 may be scheduled for routine maintenance and service (block 714). Routine maintenance and service may include modification, reconfiguration, refurbishment, etc. of one or more systems of aircraft 800.

Each of the processes of the service method 700 may be performed or carried out by a system integrator, a third party, and/or an operator (e.g., a customer). For the purposes of this description, a system integrator may include, without limitation, any number of aircraft manufacturers and major-system subcontractors; a third party may include, without limitation, any number of vendors, subcontractors, and suppliers; and an operator may be an airline, leasing company, military entity, service organization, and so on.

As shown in Fig. 8, aircraft 800 produced by the service method 700 may include airframe 802 with a plurality of high-level systems 804 and interior 806. Examples of high-level systems 804 include one or more of propulsion system 808, electrical system 810, hydraulic system 812, and environmental system 814. Any number of other systems may be included. Although an aerospace example is shown, the principles disclosed herein may be applied to other industries, such as the automotive industry. Accordingly, in addition to aircraft 800, the principles disclosed herein may apply to other vehicles, e.g., land vehicles, marine vehicles, space vehicles, etc.

The disclosed methods for manufacturing reference standards may be employed during any one or more of the stages of the manufacturing and service method 700. For example, components or subassemblies corresponding to component and subassembly manufacturing (block 706) may be fabricated or manufactured in a manner similar to components or subassemblies produced while aircraft 800 is in service (block 712). Also, one or more examples of the system(s), method(s), or combination thereof may be utilized during production stages (block 706 and block 708), for example, by substantially expediting assembly of or reducing the cost of aircraft 800. Similarly, one or more examples of the system or method realizations, or a combination thereof, may be utilized, for example and without limitation, while aircraft 800 is in service (block 712) and/or during maintenance and service (block 714).

The described features, advantages, and characteristics of one example may be combined in any suitable manner in one or more other examples. One skilled in the relevant art will recognize that the examples described herein may be practiced without one or more of the specific features or advantages of a particular example. In other instances, additional features and advantages may be recognized in certain examples that may not be present in all examples. Furthermore, although various examples of reference standards 10, 10' and 10" and methods 100, 200, 300, 500 for manufacturing such reference standards 10, 10' and 10" have been shown and described, modifications may occur to those skilled in the art upon reading the specification. The present application includes such modifications and is limited only by the scope of the claims.

The disclosure comprises the following clauses:
1. A method for manufacturing a reference standard comprising a known defect, the method comprising:
   positioning a predetermined quantity of a contaminant material having a predetermined composition between a first bonding surface of a first metallic body and a second bonding surface of a second metallic body; and
   diffusion bonding the first bonding surface of the first metallic body to the second bonding surface of the second metallic body to yield a bonded article.
2. The method of Clause 1 wherein the contaminant material comprises a contaminant material density and the first metallic body comprises a bulk density, and wherein the contaminant material density is substantially less than the bulk density.
3. The method of Clause 1 or 2 wherein the contaminant material comprises a carbonaceous material.
4. The method of any of Clauses 1 to 3 wherein the contaminant material comprises graphite.
5. The method of any of Clauses 1 to 4 wherein the first metallic body comprises a titanium alloy, wherein the second metallic body comprises the titanium alloy, and wherein the contaminant material comprises graphite.
6. The method of any of Clauses 1 to 5 wherein the contaminant material comprises a contaminant material density and the first metallic body comprises a bulk density, and wherein the contaminant material density is substantially greater than the bulk density.
7. The method of any of Clauses 1 to 6 wherein the contaminant material comprises at least one of niobium, molybdenum, tantalum, tungsten, and rhenium.
8. The method of any of Clauses 1 to 7 wherein the contaminant material comprises tungsten.
9. The method of any of Clauses 1 to 8 wherein the first metallic body comprises a titanium alloy, wherein the second metallic body comprises the titanium alloy, and wherein the contaminant material comprises tungsten.
10. The method of any of Clauses 1 to 9 wherein the positioning the predetermined quantity of the contaminant material comprises positioning the predetermined quantity of the contaminant material at a predetermined location between the first bonding surface of the first metallic body and the second bonding surface of the second metallic body.
11. The method of any of Clauses 1 to 10 further comprising fabricating at least one of the first metallic body and the second metallic body from a metallic powder feedstock by metal injection molding.
12. The method of any of Clauses 1 to 11 further comprising forming a recess in the first bonding surface of the first metallic body, wherein the positioning the predetermined quantity of the contaminant material comprises positioning the predetermined quantity of the contaminant material in the recess.
13. The method of any of Clauses 1 to 12 wherein the first bonding surface and the second bonding surface are configured to meet in intimate contact.
14. The method of any of Clauses 1 to 13 wherein, after the diffusion bonding, processing the bonded article.
15. The method of Clause 14 wherein the processing comprises at least one of hot isostatic pressing, cold isostatic pressing, machining, heat-treating, and plating.
16. The method of any of Clauses 1 to 15 further comprising repeating the positioning and the diffusion bonding to manufacture a plurality of reference standards, each reference standard of the plurality of reference standards comprising a unique known defect.
17. A reference standard for use in nondestructive inspection, the reference standard comprising:
   a first metallic body;
   a second metallic body diffusion bonded to the first metallic body along a seamless interface; and
   a predetermined quantity of a contaminant material having a predetermined composition positioned at a predetermined location proximate the seamless interface.
18. The reference standard of Clause 17 wherein the contaminant material is completely surrounded by the first metallic body and the second metallic body.
19. The reference standard of Clause 17 or 18 wherein the first metallic body comprises a titanium alloy, wherein the second metallic body comprises the titanium alloy, and wherein the contaminant material comprises graphite.
20. A method for manufacturing a reference standard comprising a known defect, the method utilizing a first metallic body comprising a first bonding surface and a second metallic body comprising a second bonding surface, the method comprising:
   forming a recess in the first bonding surface of the first metallic body;
   positioning the second bonding surface of the second metallic body in intimate contact with the first bonding surface; and
   diffusion bonding the first bonding surface of the first metallic body to the second bonding surface of the second metallic body to yield a bonded article.
21. The method of Clause 20 wherein the forming the recess comprises forming a recess having a predefined size.
22. The method of Clause 20 or 21 wherein the forming the recess comprises forming a recess having a predefined shape.
23. The method of any of Clauses 20 to 22 wherein the forming the recess comprises forming the recess at a predetermined location on the first bonding surface of the first metallic body.
24. The method of any of Clauses 20 to 23 wherein the forming the recess comprises forming a recess having a maximum width and a maximum depth, and wherein at least one of the maximum width and the maximum depth falls within a range spanning from 0.001 inches to 0.060 inches.
25. The method of any of Clauses 20 to 24 wherein the forming the recess comprises at least one of drilling the first metallic body, punching the first metallic body, and scratching the first metallic body.
26. The method of any of Clauses 20 to 25 further comprising fabricating at least one of the first metallic body and the second metallic body from a metallic powder feedstock by metal injection molding.
27. The method of any of Clauses 20 to 26 further comprising positioning a predetermined quantity of a contaminant material having a predetermined composition within said recess.
28. The method of Clause 27 wherein the contaminant material comprises a contaminant material density and the first metallic body comprises a bulk density, and wherein the contaminant material density is substantially less than the bulk density.
29. The method of Clause 27 wherein the contaminant material comprises a carbonaceous material.
30. The method of Clause 27 wherein the contaminant material comprises graphite.
31. The method of any of Clauses 27 to 30 wherein the first metallic body comprises a titanium alloy, wherein the second metallic body comprises the titanium alloy, and wherein the contaminant material comprises graphite.
32. The method of any of Clauses 27 to 31 wherein the contaminant material comprises a contaminant material density and the first metallic body comprises a bulk density, and wherein the contaminant material density is substantially greater than the bulk density.
33. The method of any of Clauses 27 to 32 wherein the contaminant material comprises at least one of niobium, molybdenum, tantalum, tungsten, and rhenium.
34. The method of any of Clauses 27 to 33 wherein the contaminant material comprises tungsten.
35. The method of any of Clauses 27 to 34 wherein the first metallic body comprises a titanium alloy, wherein the second metallic body comprises the titanium alloy, and wherein the contaminant material comprises tungsten.
36. The method of any of Clauses 27 to 35 wherein the contaminant material substantially fills the recess.
37. The method of any of Clause 20 to 36 wherein, after the diffusion bonding, processing the bonded article.
38. The method of Clause 37 wherein the processing comprises at least one of hot isostatic pressing, cold isostatic pressing, machining, heat-treating, and plating.
39. The method of any of Clauses 20 to 38 further comprising repeating the forming, the positioning, and the diffusion bonding to manufacture a plurality of reference standards, each reference standard of the plurality of reference standards comprising a unique known defect.
40. A reference standard for use in nondestructive inspection, the reference standard comprising:
   a first metallic body;
   a second metallic body diffusion bonded to the first metallic body along a seamless interface; and
   a recess positioned at a predetermined location proximate the seamless interface.
41. The reference standard of Clause 40 wherein the recess is completely surrounded by the first metallic body and the second metallic body.
42. The reference standard of Clause 40 or 41 further comprising a contaminant material having a predetermined composition received in the recess.
43. A method for manufacturing a reference standard comprising a known defect, the method comprising:
   incorporating a predetermined quantity of a contaminant material having a predetermined composition into a metallic powder composition to yield a contaminated metallic powder composition;
   shaping the contaminated metallic powder composition to yield a shaped metallic powder composition; and
   sintering the shaped metallic powder composition to yield a consolidated article.
44. The method of Clause 43 wherein the incorporating the predetermined quantity of the contaminant material into the metallic powder composition comprises incorporating the predetermined quantity of the contaminant material into a predetermined quantity of the metallic powder composition to yield the contaminated metallic powder composition having a known contaminant material concentration.
45. The method of Clause 43 or 44 wherein the incorporating the predetermined quantity of the contaminant material into the metallic powder composition comprises dispersing the predetermined quantity of the contaminant material throughout the metallic powder composition.
46. The method of any of Clauses 43 to 45 wherein the contaminant material comprises a contaminant material density and the metallic powder composition comprises a bulk density, and wherein the contaminant material density is substantially less than the bulk density.
47. The method of any of Clauses 43 to 46 wherein the contaminant material comprises a carbonaceous material.
48. The method of any of Clauses 43 to 47 wherein the contaminant material comprises graphite.
49. The method of any of Clauses 43 to 48 wherein the metallic powder composition comprises a titanium alloy, and wherein the contaminant material comprises graphite.
50. The method of any of Clauses 43 to 49 wherein the contaminant material comprises a contaminant material density and the metallic powder composition comprises a bulk density, and wherein the contaminant material density is substantially greater than the bulk density.
51. The method of any of Clauses 43 to 50 wherein the contaminant material comprises at least one of niobium, molybdenum, tantalum, tungsten, and rhenium.
52. The method of any of Clauses 43 to 51 wherein the contaminant material comprises tungsten.
53. The method of any of Clauses 43 to 52 wherein the metallic powder composition comprises a titanium alloy, and wherein the contaminant material comprises tungsten.
54. The method of any of Clauses 43 to 53 wherein the shaping the contaminated metallic powder composition comprises injecting the contaminated metallic powder composition into a mold cavity to yield the shaped metallic powder composition.
55. The method of Clause 54 wherein the contaminated metallic powder composition comprises a binder in admixture with the contaminant material and a metallic powder.
56. The method of Clause 54 or 55 further comprising heating the contaminated metallic powder composition during the shaping.
57. The method of any of Clauses 54 to 56 further comprising debinding the shaped metallic powder composition prior to the sintering.
58. The method of any of Clauses 43 to 57 wherein, after the sintering, processing the consolidated article.
59. The method of Clause 58 wherein the processing comprises at least one of hot isostatic pressing, cold isostatic pressing, machining, heat-treating, and plating.
60. The method of any of Clauses 43 to 59 further comprising repeating the incorporating, the shaping, and the sintering to manufacture a plurality of reference standards, each reference standard of the plurality of reference standards comprising a unique known defect.
61. A reference standard manufactured according to the method of any of Clauses 43 to 60.

## Claims

1. A method (100) for manufacturing a reference standard (10) comprising a known defect (12), the method comprising:
positioning (130) a predetermined quantity of a contaminant material (14) having a predetermined composition between a first bonding surface (22) of a first metallic body (20) and a second bonding surface (32) of a second metallic body (30); and
diffusion bonding (140) the first bonding surface (22) of the first metallic body (20) to the second bonding surface (32) of the second metallic body (30) to yield a bonded article (40).

2. The method (100) of Claim 1 wherein the contaminant material (14) comprises a contaminant material density and the first metallic body (20) comprises a bulk density, and wherein the contaminant material density is substantially less than the bulk density.

3. The method (100) of Claim 1 or 2 wherein the contaminant material (14) comprises a carbonaceous material; or
wherein the contaminant material (14) comprises graphite.

4. The method (100) of any preceding Claim wherein the first metallic body (20) comprises a titanium alloy, wherein the second metallic body (30) comprises the titanium alloy, and wherein the contaminant material (14) comprises graphite.

5. The method (100) of Claim 1, 3 or 4 wherein the contaminant material (14) comprises a contaminant material density and the first metallic body (20) comprises a bulk density, and wherein the contaminant material density is substantially greater than the bulk density.

6. The method (100) of any preceding Claim wherein the contaminant material (14) comprises at least one of niobium, molybdenum, tantalum, tungsten, and rhenium; or
wherein the contaminant material (14) comprises tungsten.

7. The method (100) of any preceding Claim wherein the first metallic body (20) comprises a titanium alloy, wherein the second metallic body (30) comprises the titanium alloy, and wherein the contaminant material (14) comprises tungsten.

8. The method (100) of any preceding Claim wherein the positioning the predetermined quantity of the contaminant material (14) comprises positioning the predetermined quantity of the contaminant material (14) at a predetermined location (L) between the first bonding surface (22) of the first metallic body (20) and the second bonding surface (32) of the second metallic body (30).

9. The method (100) of any preceding Claim further comprising fabricating (110) at least one of the first metallic body (20) and the second metallic body (30) from a metallic powder feedstock (502) by metal injection molding (500).

10. The method (100) of any preceding Claim further comprising forming (120) a recess (24) in the first bonding surface (22) of the first metallic body (20), wherein the positioning (130) the predetermined quantity of the contaminant material (14) comprises positioning the predetermined quantity of the contaminant material (14) in the recess (24).

11. The method (100) of any preceding Claim wherein the first bonding surface (22) and the second bonding surface (32) are configured to meet in intimate contact.

12. The method (100) of any preceding Claim wherein, after the diffusion bonding (140), processing (150) the bonded article (40); and optionally
wherein the processing (150) comprises at least one of hot isostatic pressing, cold isostatic pressing, machining, heat-treating, and plating.

13. The method (100) of any preceding Claim further comprising repeating (160) the positioning (130) and the diffusion bonding (140) to manufacture a plurality of reference standards (10), each reference standard (10) of the plurality of reference standards (10) comprising a unique known defect (12).

14. A reference standard (10) for use in nondestructive inspection, the reference standard (10) comprising:
a first metallic body (20);
a second metallic body (30) diffusion bonded to the first metallic body (20) along a seamless interface (42); and
a predetermined quantity of a contaminant material (14) having a predetermined composition positioned at a predetermined location (L) proximate the seamless interface (42).

15. The reference standard (10) of Claim 15 wherein the contaminant material (14) is completely surrounded by the first metallic body (20) and the second metallic body (30) and/or
wherein the first metallic body (20) comprises a titanium alloy, wherein the second metallic body (30) comprises the titanium alloy, and wherein the contaminant material (14) comprises graphite.
